# NEW EUROPEAN PATENT SPECIFICATION

(11) **EP 1 554 987 B2**
(45) Date of publication and mention of the opposition decision: **02.12.2015**
(45) Mention of the grant of the patent: 25.03.2009
(21) Application number: 03753604.2
(22) Date of filing: 01.10.2003
(51) Int. Cl.: A61B 19/00, A61B 6/00

(54) **FUNCTIONAL NAVIGATOR**
FUNKTIONSNAVIGATOR
NAVIGATEUR FONCTIONNEL

(30) Priority: 01.10.2002 ES 200202220
(43) Date of publication of application: 20.07.2005
(73) Proprietor: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: BENLLOCH BAVIERA, J.M., CSIC - Univ. de Valencia, 46100 Burjassot (Valencia) (ES); ALCANIZ RAYA, M., Univ. Politecnica de Valencia, 46022 Valencia (ES); SANCHEZ MARTINEZ, F., CSIC - Univ. de Valencia, 46100 Burjassot (Valencia) (ES); GRAU COLOMER, V., Univ. Poilitecnica de Valencia, 46022 Valencia (ES)
(74) Representative: Grünecker Patent- und Rechtsanwälte PartG mbB
(86) International application number: PCT/ES2003/000497
(87) International publication number: WO 2004/030561

(56) References cited:
- WO-A-01/76497
- WO-A-01/79884
- WO-A2-01/37748
- WO-A2-01/79884
- WO-A2-02/060316
- DE-A- 19 639 615
- DE-A1- 10 037 491
- US-A- 6 165 181
- US-A- 2001 029 333
- US-A1- 2001 029 333
- US-A1- 2002 042 566
- US-A1- 2002 095 081
- US-A1- 2002 103 431
- SCHULDER M. ET AL: 'Cranial surgery navigation aided by a compact intraoperative magnetic resonance imager' J. NEUROSURG vol. 94, June 2001, pages 936 - 945
- SEIFERT V.: 'Intraoperative MRI in neurosurgery: Technical overkill or the future of brain surgery?' NEUROLOGY INDIA vol. 51, no. 3, 2003, pages 329 - 332
- Declaration under 37 C.F.R. § 1.132 of Benlloch Baviera, Jose Maria, et al.
- Image registration. From Wikipedia, the free encyclopedia

## Description

### TECHNICAL FIELD OF THE INVENTION

The invention comes within the sector of Medicine, and in particular Nuclear Medical Physics. It is a device which permits the precise location and extirpation of carcinogenic tumours at any point of the human body

### BACKGROUND OF THE INVENTION

Cancer is a disease that is produced as a result of an uncontrolled development of abnormal or defective cells, which tend to infiltrate, spread and metastasise, or invade, other tissues, whether surrounding or distant from the organ where the disease started. A series of diagnostic techniques and therapeutic methods are currently available which specialists use for the diagnosis and treatment of cancer.

Among the different diagnostic techniques, specialists use image diagnosis systems. These systems offer morphological images (CAT, Magnetic resonance, Mammography, etc.) or functional images (Gamma camera, PET camera). The most advanced diagnostic techniques use this type of image since they show the carcinogenic activity of the zone independently of its shape, size, etc., which can always give rise to erroneous diagnoses. The morphological images analyse the structure of the organs to study while functional images provide information on metabolic processes at the cellular level of those same organs. In many cases, an apparently normal structure of an organ or tissue hides an abnormal functioning which can be detected by means of functional images of the biological processes.

When establishing the diagnosis of the cancer, the local extension of the disease and the neighbouring structures that might have become involved must be known. Knowing the presence or absence of regional or distant metastasis is essential for treatment of cancer.

For this reason, when a carcinogenic tumour is extirpated, a very large number of ganglia to be found in the vicinity of that tumour are also removed if there is any suspicion that they might be carcinogenic. Those ganglia are then sent to the pathological anatomy laboratory for analysis. In most cases just a very small number (less than 5%) of ganglia turn out to be damaged.

For the detection of infected ganglia there exists a technique, which is carried out intra-operatively, and which consists of detecting carcinogenic ganglia by means of probes which emit sound of greater intensity the closer the probe approaches an inflamed ganglion (Intraoperative Lymphatic Mapping and Sentinel Lymph Node Dissection in Breast Cancer (2000), CA Cancer J Clin 2000; 50: 279-291, E. C. Hsueh; N. Hansen, A. E. Giuliano). Nevertheless, this technique is somewhat rudimentary since it does not allow the ganglia to be located accurately and it is not applicable when the ganglia are to be found at a certain depth from the surface of the patient.

The most advanced technique at present for identifying infected ganglia consists of using Gamma cameras which form a functional image of the zone to examine and permit nearby ganglia to be distinguished from each other (High-resolution Hand-held Gamma Camera, L. MacDonald et al.; Proc. SPIE Vol., 4142, p. 242-253, Penetrating Radiation Systems and Applications II, F. Patrick Doty; H. Bradford Barber; Hans Roehring; Edward J., Morton; Eds. 12/2000). The problem of this technique is that the images obtained are two-dimensional projections of the field of view of the camera and in many cases it is not easy to locate the depth of a particular ganglion that is inflamed. Also, there does not exist any correspondence in the image with the surgeon's surgical instruments (Dynamic Sentinel Node Biopsy for Penile Cancer; Reliability of a Staging Technique, The Journal of Urology 2002;168:76-80 P. J. Tanis, A. P. Lont; W. Meinhardt; R. A. Valdés Olmos; O. E. Nieweg; S. Horenblas).

On the other hand, mainly in the field of neurosurgery, increasing use is being made of what are known as navigators (Taylor, R., Lavelée, S., Burdea, G., Mösges ed., Computer Integrated Surgery; Technology and Clinical Applications, MIT Press, Cambridge, MA, 1996); (Laborde, G., Gilsbach, J., Harders, A., Moesges, R. and Krybus, W., Computer assisted localiser for planning of surgery and intra-operative orientation, Acta Neurochir., 119:166-170 (1992)); (Troccaz, J., Grimson, E., and Mösges, R. ed., CVRMed-MRCAS'97 First joint conference on computer vision, virtual reality and robotics in Medicine, and Medical robotics and Computer-assisted surgery Grenoble Mar 19-23, Proceedings, Springer, 1997); (The Journal of Image Guided Surgery (or under its new name Computer Aided Surgery). This magazine is the voice of "ISCAS", the international society for computer aided surgery); (Nolte, L-P, Zamorano, L., Jiang, Z., Wang, Q., Langlotz, F. and Berlemann, U. Image-guided insertion of transpedicular screws., Spine 20:497-500 (1995)); (Watanabe, E., Watanabe, T., Manaka, S., Mayanagi, Y. and Takakura., K. Three dimensional digitizer (Neuronavigator): New equipment for computed tomography-guided stereotaxic surgery. Surg. Neurol. 24:543-7 (1987)); (Rohling, R., Munger, P., Hollerbach, J. and Peters, T., Comparison of relative accuracy between a mechanical and an optical position tracker for image-guided neurosurgery, J. Image Guided Surg., 1:30-34 (1995)); (Troccaz, J., Grimson, E. and Mösges, R. ed., CVRMed-MRCAS'97: First Joint Conference on Computer Vision, Virtual Reality and Robotics in Medicine, and Medical Robotics and Computer Assisted Surgery, Grenoble, France, March 19-22, 1997, Springer (Berlin)); (American Soc. for Testing and Materials (ATSM), Standard Specification for Image-Interactive Stereotactic and Localization Systems, F1719-96, Oct. 1996); (American Soc. for Testing and Materials (ATSM), Performance specification for cerebral stereotactic instruments, F1266, Annual book ofd ASTM Standards, Vol 13.01). Navigators are extraordinarily useful devices in intra-operative work since they permit surgical instruments (scalpels, clamps, etc.) to be located with a high degree of precision with regard to the patient's organs or tumours by comparing them with a CAT or Magnetic Resonance image taken prior to the operation.
Apart from the above mentioned navigators based on structural images it has been found a navigator system based on functional images in publication WO 01/079884 A3. In this invention, and differently from the above-mentioned navigators, tomographic functional image equipment is used instead of 3D structural information in order to generate the navigation images. Hence, previously acquired 3D functional images are used, which are equivalent to the structural images with respect to their applicability during navigation, since they are used to guide the surgical instruments during the intervention. We emphasise that the use of functional images in application WO 01/079884 A3 is equivalent to the use of structural images in other equipment of the state of the art. The only difference is that in the later ones the guiding is performed following the anatomic structure whilst in the former it is performed following the functional response of the organism under study.
It is very important to consider that in both cases the information is obtained previously to the intervention and hence the surgeon is not able to check in real time (during the surgery) the effectiveness of his intervention, for instance, whether there are some cancerous tissues still in the patient that have not yet been excised. It is also important to realise that in the case of the equipment based on functional images it is requested that the functional images must be tomographic or fully 3D. There is no navigator that allows 3D navigation using planar functional images.
It has to be also pointed out that to this moment there are no navigators that obtain simultaneous information of both structural and functional images in real time.
The image information used by current surgical navigators is, as mentioned above, either purely 3D morphological (from magnetic resonance images (MRI) or X-ray CT), or purely 3D functional (from gamma tomographic images). Moreover, the information is obtained previously to the intervention, and therefore the surgeon is not able to check in real time (at the surgery) the effectiveness of his intervention, whether there are some cancerous tissues still in the patient that have to be removed.

So, there does not currently exist instruments permitting the ganglia and tumours to be precisely located at the moment of the operation.

With the present invention, which we call "Functional Navigator", the aim is to develop a set of equipment that will permit the ganglia and tumours to be located exactly and three-dimensionally intra-operatively and to correlate the surgical instruments with the images in real time.

Patent Application US 2001/0029333 A1, describes an equipment which makes use of a standard navigator with a previously obtained morphological image, and which is used in combination with another system of morphological image, but which could be used in real time in order to precise and to correlate both 3D images. In this way, an image is formed starting from the point of view of the real time image system, and to the extent that this system moves into the interior of the observed region or in contact with the exterior surface of the observed volume. [0017;0018; 0048], (this obstacle hinders the simultaneous use of surgical instruments in the same region. This requires the acquisition of several images at different depths or directions [0016].
This system is oriented towards generating a 3D structural image in real time from the point of view and orientation of the surgical instruments [0017; 0020]; or a combined image that uses the previous structural 3D image of the region to be observed and the instantaneous structural image from an ultrasound equipment, endoscope or microscope, that serves also as an instantaneous geometric reference image fusion presented [0018; 0024]. It is an important limitation that the real time image provided by this equipment is only of morphological nature, without including functional information of the observed region, which limits its capabilities to allowing a finer positioning and, over all, confirms in real time what it is already known from the previous morphological image.
It is important to point out that the need of performing rotations adds an extra difficulty to the system, since it requires a continuous positioning of the ultrasound equipment in each of the angles and its final superposition of all the images obtained at different angles to generate the mentioned 3D view, with the difficulty that in all these rotations the ultrasound equipment positioning systems must be visible for the navigation localizer, which could present practical difficulties to the use of the system from D2.

### DESCRIPTION OF THE INVENTION

The object of the invention is a surgical navigator which uses information on the basis of three-dimensional functional images obtained in real time. The functional information is mainly obtained from Gamma cameras or PET cameras.

The most advanced current techniques for locating tumours intra-operatively consist of using Gamma mini-cameras which provide functional images. The problem of this technique is that the images obtained are two-dimensional projections of the field of view of the camera and in many cases it is not easy to locate the depth of a particular ganglion that is inflamed. Also, there does not exist any correspondence in the image with the surgeon's surgical instruments.

The functional navigator forming the object of the invention, which is as defined in the appended set of claims is essentially characterised in that the position of the cameras which acquire the functional images is controlled by the navigator, for example by means of emitters or reflectors (basically infrared or magnetic) provided in them. The information from the images of the cameras and the position of them, determined by the navigator, is analysed by a specific software program and combined with the position of the surgical instruments, which is also monitored by the navigator, in order to obtain images. Therefore, in the present invention, the camera is coupled to the navigator so that they can act jointly and in coordination.

A particular object of the present invention is the use of the surgical navigator for the positive and precise detection of carcinogenic tumours in surgery and its combination with the surgical instruments.

The obtaining of functional images by means of a Gamma or PET camera during the surgical operation contributes essential information for deciding on the area to cut out during the actual moment of the operation and for checking that a tumour has been removed correctly and in its entirety. The present invention has applications not just in the field of oncology but also in neurosurgery and cardiology.

In detail, the object of the invention is a surgical navigator which uses information on the basis of three-dimensional functional images obtained in real time. A functional image is understood as being that which shows the metabolic activity of the cells of the organs or tissues to be studied. The functional information is mainly obtained from Gamma cameras or PET (Positron Emission Tomography) cameras.

The surgical navigator is essentially characterised in that the position of the cameras which acquire the functional images is controlled by the navigator, for example by means of emitters or reflectors basically of infrared or magnetic, provided in the cameras. The information from the images of the cameras and the position of them, determined by the navigator, is analysed by a specific software program and combined with the position of the surgical instruments, which is also monitored by the navigator, in order to obtain images.

In the present invention, use is made of the diagnostic methods of nuclear medicine, in which a type of contrast is first introduced in the patient. Once the contrast has spread through the patient's organs, these organs are examined by means of a gamma ray detection camera (Gamma camera or PET camera). The process is similar to taking a photograph of the patient except that gamma rays are used which have a wavelength far less than that of visible light, providing information on the region where the contrast has accumulated.

The camera is coupled to the navigator so that they can act jointly and in coordination. The camera provides functional information in real time which can be combined by means of a specific software program with the morphological information possessed by the navigator in order to form a complete morphological and functional image, which is of extraordinary utility for the surgeon.

In order to combine the two types of image correctly, the navigator needs to have precise information on the location of the portable camera at each moment. For this, a special system has been designed for location of the camera. The position of the camera is determined by the navigator via the infrared emitters. A specific software program combines the images obtained by the camera in different positions in order to form at least one stereoscopic image of an organ.

The type of image that is obtained presents at least stereoscopic information on the desired object by means of a pair of projections separated by about 90°. In the case in which quasi-point objects are examined, such as ganglia, the stereoscopic information is sufficient for locating them precisely in three dimensions. In the case of extensive objects, such as an organ or a tumour, more projections in space are acquired in order to obtain a tomographic image of them.

The infrared emitters are also located in the surgical instruments and are detected by the navigator as in a normal system. The difference here is that the surgeon obtains functional information on-line.

During the surgery, the quantity of radiation required by the Functional Navigator with Gamma cameras for being introduced into the patient is around 200 µCi. This dose is sufficiently high for rapidly visualising an affected ganglion since it is injected into the region to study and it accumulates in the volume of the ganglion which is virtually a point (minimum of 5 mm in diameter). On the other hand, this quantity of activity is not so great as to affect the medical team carrying out the surgery which, as a precaution, can wear lead gowns.

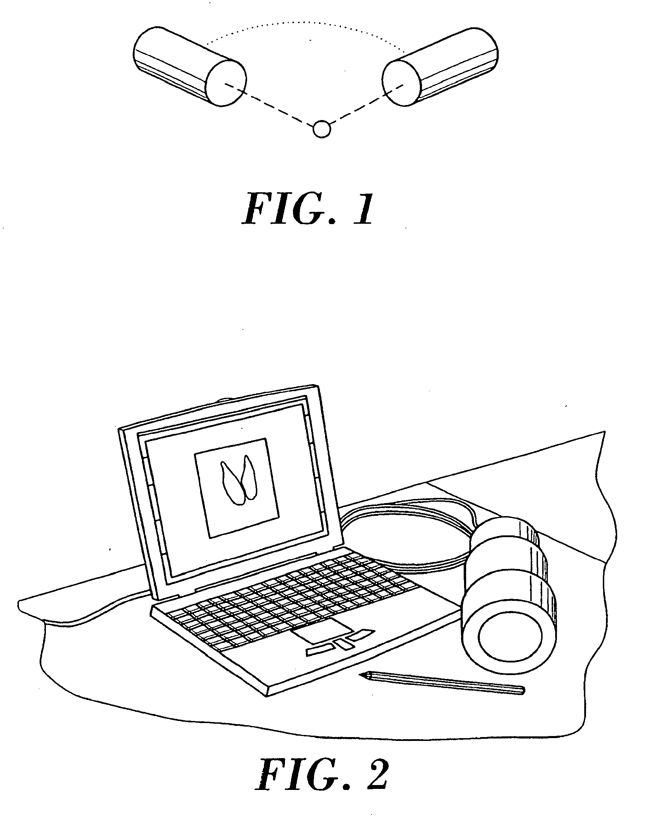

The position of the ganglion is determined by triangulation, taking the intersection of the lines determined by the camera from two different positions separated by 90° (see figure 1) or three, separated by 120° (surrounding the patient). Two different lead collimators have been used for the Gamma cameras: a pinhole and a parallel ray collimator. If the region under study is limited, the parallel ray one is used, since its resolution is better and independent of the distance to the ganglion (unlike in the case of the pinhole). Obviously, in either of the cases, the camera has to be located as close as possible to the region under study in order to increase the sensitivity and improve the spatial resolution.

Various laboratory tests have been developed with phantoms full of technetium and the results obtained are as follows: the position of the simulated sentinel ganglion is determined by the instrument of the navigator with an error of less than 3 mm. This error is sufficiently small for distinguishing any affected ganglion from its healthy neighbours.

In the case of the functional navigator with PET cameras, the dose introduced in the patient is around 1 mCi. Owing to the fact that the energy of the gamma rays when PET cameras are used is greater than when Gamma cameras are used, lead gowns are not sufficient for providing protection against radiation. In this case, leaded glass screens need to be used or surgical robots remotely controlled by the surgeon can be employed.

A Gamma and PET camera currently provide an image in two dimensions. In order to obtain three-dimensional images, two different methods are used: a single camera which acquires images in two different planes (for example, locating the portable camera in different positions at 90° with respect to each other) in order to thereby obtain a stereoscopic image; or two cameras joined by means of a mechanical system in order to keep them at a fixed angle with regard to each other but variable with regard to the axis of the patient. This latter model is faster but implies a higher cost of the equipment.

Once the image has been captured, it is processed by special software specifically developed for this system. This software is a computing program for analysis which combines the information from the cameras with the navigator. In the case of ganglia, a software program has been developed which locates them on the basis of the images obtained from the camera in different positions.

Finally, a navigator is obtained which has functional information with stereoscopic vision available at the moment the exploration is being conducted. The image in three dimensions or in various planes of the object to study is presented on screen together with the surgical material.

On account of everything stated, the structural elements of the functional navigator are: a surgical navigator, a camera which obtains functional images, the position-finding elements of the camera and the surgical instruments and the specific software program which combines all of the information from the aforementioned elements.

The cameras used in this invention are, as has been said above, Gamma cameras or PET cameras, or any type of camera that has been adapted for obtaining this type of image. These cameras have to permit access to the patient by the surgical instruments and for this reason the Gamma or PET cameras used in the invention have been designed to be small and portable.

A particular object of the invention is the use of the functional navigator for the positive and precise detection of carcinogenic tumours in the operating theatre and its combination with the surgical instruments.

The obtaining of functioning images by means of a Gamma or PET camera during the surgical operation provides essential information for deciding on the area to cut out during the actual moment of the operation and checking whether a tumour has been removed correctly and in its entirety. Also, the functional navigator solves the problem of movements of the patient's organs during the time that passes from taking the images to the surgical operation, so there is greater security in extraction of, for example, carcinogenic cells during the surgery.

Another particular and additional object of the invention is its use for the positive and precise detection of carcinogenic ganglia in the operating theatre and its combination with the surgical instruments.

The functional navigator with Gamma cameras will fundamentally be used for the detection of carcinogenic ganglia in oncology, though it could also have applications in neurology and cardiology. The functional navigator with PET cameras will essentially be used for the location of tumours in oncology, though it too could be used in neurology in, for example, the location and correction of foci of epilepsy and it could also have applications in cardiology.

So, this invention is useful for any radical surgery.

In the case of extirpation of ganglia, the surgical procedure could be known as "*lymphanectomy guided by gamma-camera (LGG)*".

Lymphanectomy guided by gamma-camera would be a minimally invasive surgery having various benefits for the patient:
- It decreases the extent of the surgery, saving time, costs and any complications deriving from an aggressive extirpation of ganglia (lymphoceles, lymphatic oedema, etc.) .
- It saves the benign ganglia, permitting them to continue with their immunological task. Its applications will fundamentally be focused on the field of oncology.

### BRIEF DESCRIPTION OF THE FIGURES

Figure 1: diagram of the two different positions we use for locating the ganglion
Figure 2: photograph of the Gamma mini-camera operating from a portable computer via a USB port
Figure 3: photograph of the "functional navigator": it shows the interferometer oriented towards the infrared emitter pellets located in the camera and in the surgical instrument
Figure 4: example of functional navigator screen with Gamma cameras where the ganglia appear
Figure 5: example of functional navigator screen with Gamma cameras where the cross represents the location of the surgical instruments

### DESCRIPTION OF EMBODIMENTS OF THE INVENTION

### Example 1: Embodiment of the functional navigator with Gamma cameras

For this, a description is given first of all of a Gamma mini-camera and then of the surgical navigator used.

The Gamma mini-camera has a total size of diameter 90 mm and length 200 mm, and a total weight of somewhat less than 2 kg. The two characteristics, small dimensions and light weight, ensure mobility of the system (see figure 2). The Gamma mini-camera has a spatial resolution of about 2 mm. This Gamma camera is used for visualising small organs.

The design of a portable Gamma mini-camera is optimised for the radioactive source most widely used in medical explorations, that of 99m-Tc, which emits gamma rays of 140 keV. The camera consists of a single position-sensitive photomultiplier tube (PSPMT, HR2486 (from Hamamatsu Photonica)) coupled to a scintillation crystal, an electronic system and a high voltage source, with lead collimators being able to be coupled easily.

Physical measurements have been carried out in accordance with industrial requirements, specified by the National Electrical Manufacturers Association (NEMA) in order to evaluate the functioning of the camera. The NEMA standards for evaluating the characteristics of the camera were established for conventional equipment, and they therefore had to be adapted to the size and operating conditions of the system forming the object of the invention. These measurements were made with sources of 99m-Tc with the required activities. These are point sources of diameter 2 mm, capillary tube of 2 mm and plastic petri dish of diameter 6 cm, which provides a uniform flow. The characteristics of the camera are: extrinsic spatial resolution of 3 mm (camera with collimator), energy resolution of 12.8% at 140 keV, planar sensitivity of 120 cpm/mCi (according to the geometric efficiency of the collimator) and a field of view (FOV) of 46 mm. These values permit small organs to be visualised, such as the thyroid, kidney and sentinel ganglia.

A clinical test conducted with a thyroid phantom using 99m-Tc and a total activity of 200 µCi, and some clinical cases with patients, show that the system provides high quality images of a real thyroid with the usual doses (2 mCi) in about 10 minutes. The mobility and weight of the camera permit the patient's organ to be visualised in any desired direction.

On the other hand, the hardware for the surgical navigator consists of a 3D (Polaris; Northern Digital, Waterloo, ON, Canada) optical digitiser and a computer which executes our own software in C++ within a WINDOWS NT environment. The software of this system includes a surgical planning module for: image capture, segmentation, 3D visualisation and planning of surgical procedures, and an intra-operative software module which guides the surgeon by means of image in the operating theatre. This interface includes, among other things, three planes of the image, oblique reformatting and a rapid localisation of specific sources by means of a simple definition of planes. For the rapid interpretation of 3D images, a parallel implementation is used of volume interpretation algorithms, all this being executed in a network of PC processors. Ray capture techniques are used that are divided among the processors in order to obtain high quality interactive images. This novel feature provides a very useful and interactive tool for the visualisation of the patent's anatomy by means of using simple and cheap hardware solutions. A parallel version of some segmentation tools has also been implemented for semi-automatic delineation of critical structures such as vessels. The use of high performance computation permits real time desegmentation and handling of anatomical structures.

Both the Gamma camera and the surgical navigator are connected to a portable computer via a USB interface. The information from both systems is analysed and combined by a single program in the computer.

The position of the Gamma camera is determined by the navigator by means of infrared emitters, which also locate the surgical instruments. A software program combines the images obtained by the Gamma camera in different positions in order to form a stereoscopic image of an organ.

The functional navigator has been tested with the Gamma camera with different ganglia phantoms, giving very positive results in terms of the spatial resolution and separation between neighbouring ganglia.

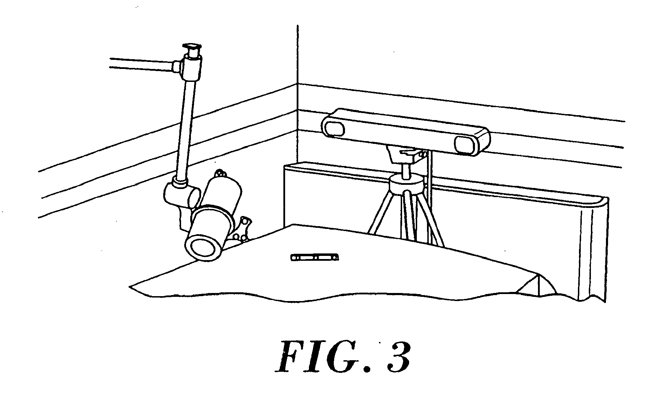

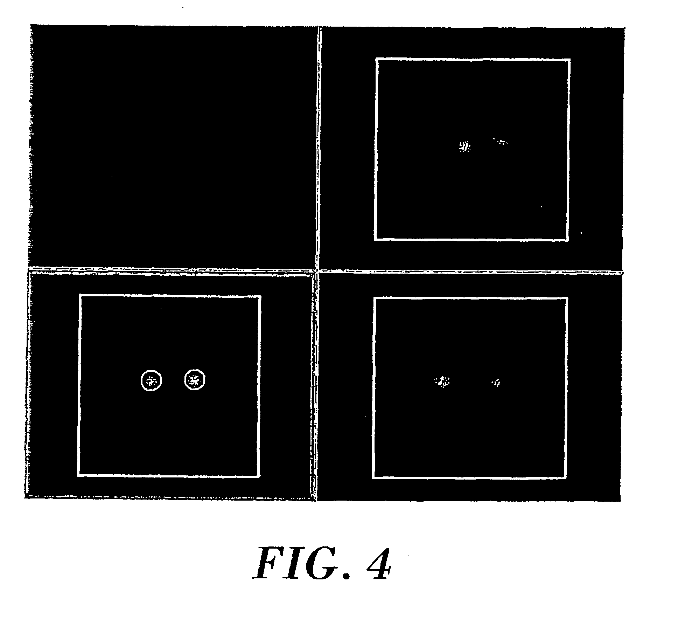

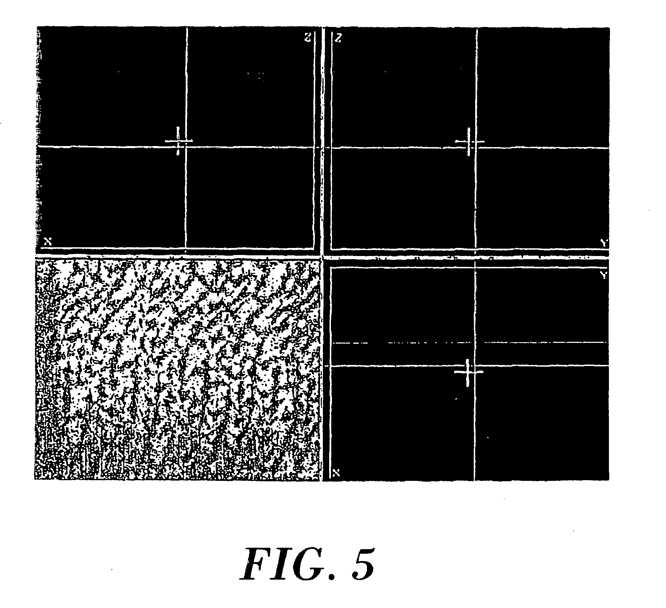

In figure 3 we can see a photo of the functional navigator with the Gamma camera. Figure 4 shows the screen of the navigator with two ganglia in three different projections and figure 5 shows the screen which guides the surgeon towards the chosen ganglia. The cross on the screen signifies the position of the scalpel.

## Claims

1. A system for coordinating a surgery process comprising:
a standard surgical navigator comprising structural 3D imaging capabilities and providing a morphological CAT or magnetic resonance image obtained before the surgery process;
a gamma camera or a plurality of gamma cameras for obtaining functional 3D images showing the metabolic activity of cells of organs or tissues in real time,
surgical instruments,
position finding elements located on said surgical instruments and said gamma camera or cameras to correlate said gamma camera or cameras and said surgical instruments with said navigator;
a specific software program which combines all information from the aforementioned elements;
wherein the functional images are processed and combined with the morphological image and used to identify different metabolic activity threshold areas of affected tissues to be monitored in order to permit the surgical instruments to be positioned with regard to said areas.

2. The system of claim 1 in which the processing of functional images is performed by triangulation, stereoscopy, or tomography.

3. The system of claim 1 or 2, **characterised in that** the functional images are acquired and processed in real time.

4. The system of any of claims 1, 2 or 3, **characterised in that** the functional images are obtained by means of PET gamma cameras or any gamma camera adapted for obtaining said type of three dimensional images.

## Patentansprüche

1. Ein System zur Koordination eines chirurgischen Vorgangs, mit:
einem chirurgischen Standard-Navigator, der Möglichkeiten zur strukturellen 3D-Bildgebung aufweist und ein morphologisches CAT-Bild oder Magnetresonanzbild, das vor dem chirurgischen Vorgang erhalten wurde, bereitstellt;
einer Gamma-Kamera oder einer Vielzahl von Gamma-Kameras zum Erhalten von funktionalen 3D-Bildern, die die metabolische Aktivität von Zellen von Organen oder Geweben in Echtzeit zeigen,
chirurgischen Instrumenten,
Positionsdetektionselementen, die auf den chirurgischen Instrumenten und der Gamma-Kamera oder den Gamma-Kameras angeordnet sind, um die Gamma-Kamera oder die Gamma-Kameras und die chirurgischen Instrumente mit dem Navigator zu korrelieren;
einem spezifischen Software-Programm, das alle Informationen aus den vorstehenden Elementen kombiniert;
wobei die funktionalen Bilder verarbeitet und mit dem morphologischen Bild kombiniert und verwendet werden, um unterschiedliche Grenzbereiche metabolischer Aktivität zu überwachender betroffener Gewebe zu identifizieren, so dass die chirurgischen Instrumente in Bezug auf die Bereiche positioniert werden können.

2. Das System nach Anspruch 1, wobei das Verarbeiten der funktionalen Bilder durch Triangulation, Stereoskopie oder Tomographie erfolgt.

3. Das System nach Anspruch 1 oder 2, **dadurch gekennzeichnet, dass** die funktionalen Bilder in Echtzeit aufgenommen und verarbeitet werden.

4. Das System nach einem der Ansprüche 1, 2 oder 3, **dadurch gekennzeichnet, dass** die funktionalen Bilder durch PET-Gamma-Kameras oder jedwede Gamma-Kamera erhalten werden, die zum Aufnehmen dieser Art dreidimensionaler Bilder eingerichtet ist.

## Revendications

1. Système de coordination d'un processus chirurgical comprenant:
un navigateur chirurgical standard comprenant des capacités de formation d'images structurelles 3D et fournissant une image CAT morphologique ou une image par résonance magnétique obtenue avant le processus chirurgical;
une caméra gamma ou une pluralité de caméras gamma pour obtenir des images fonctionnelles 3D qui montrent l'activité métabolique des cellules d'organes ou de tissus en temps réel,
des instruments chirurgicaux,
des éléments de localisation situés sur lesdits instruments chirurgicaux et ladite caméra gamma ou lesdites caméras gamma afin de corréler ladite caméra gamma ou lesdites caméras gamma et lesdits instruments chirurgicaux avec ledit navigateur et
un programme logiciel spécifique qui combine toutes les informations des éléments mentionnés ci-dessus;
dans lequel les images fonctionnelles sont traitées et combinées avec l'image morphologique et utilisées pour identifier différentes zones de seuil d'activité métabolique de tissus affectés devant être surveillés, afin de permettre de positionner les instruments chirurgicaux par rapport auxdites zones.

2. Système de la revendication 1 dans lequel le traitement des images fonctionnelles est effectué par triangulation, stéréoscopie ou tomographie.

3. Système de la revendication 1 ou 2, **caractérisé en ce que** les images fonctionnelles sont acquises et traitées en temps réel.

4. Système suivant l'une des revendications 1, 2 ou 3, **caractérisé en ce que** les images fonctionnelles sont obtenues à l'aide de caméras gamma PET ou de toute caméra gamma conçue pour obtenir ledit type d'images tridimensionnelles.
